# EUROPEAN PATENT SPECIFICATION

(11) **EP 1 279 743 B1**
(45) Date of publication and mention of the grant of the patent: **15.04.2009**
(21) Application number: 02016784.7
(22) Date of filing: 26.07.2002
(51) Int. Cl.: C12Q 1/68

(54) **Detection of genetic polymorphisms by allele specific amplification using modified primers and subsequent hybridization**
Nachweis von genetischen Polymorphismen durch allel-spezifische Amplifizierung unter Benutzung von angepassten Primern und darauffolgende Hybridisierung
Détection des polymorphismes génétiques par amplification allèle spécifique en utilisant des amorces modifiées et hybridation subséquente

(30) Priority: 26.07.2001 US 915780
(43) Date of publication of application: 29.01.2003
(73) Proprietor: DR. Chip Biotechnology Incorporation, Junan Township, Miaoli County, Taiwan 350 (TW)
(72) Inventor: Terng, Harn-Jing, c/o Dr.Chip Biotechology Inc., Hsinchu 300 (TW); Wu, Pei-Hua, c/o Dr.Chip Biotechology Inc., Hsinchu 300 (TW); Wang, Shin-Hwan, c/o Dr.Chip Biotechology Inc., Hsinchu 300 (TW)
(74) Representative: Becker Kurig Straus

(56) References cited:
- WO-A-01/36687
- WO-A-97/32040
- US-A- 5 578 458
- KUPPUSWAMY M N ET AL: "SINGLE NUCLEOTIDE PRIMER EXTENSION TO DETECT GENETIC DISEASES: EXPERIMENTAL APPLICATION TO HEMOPHILIA B (FACTOR IX) AND CYSTIC FIBROSIS GENES" PROCEEDINGS OF THE NATIONAL ACADEMY OF SCIENCES OF USA, NATIONAL ACADEMY OF SCIENCE. WASHINGTON, US, vol. 88, no. 4, 15 February 1991 (1991-02-15), pages 1143-1147, XP000371662 ISSN: 0027-8424
- GOBINDA SARKAR ET AL: "CHARACTERIZATION OF POLYMERASE CHAIN REACTION AMPLIFICATION OF SPECIFIC ALLELES" ANALYTICAL BIOCHEMISTRY, ACADEMIC PRESS, SAN DIEGO, CA, US, vol. 186, 1990, pages 64-68, XP000386280 ISSN: 0003-2697
- STETSENKO D A ET AL: "EFFICIENT CONJUGATION OF PEPTIDES TO OLIGONUCLEOTIDES BY NATIVE LIGATION" JOURNAL OF ORGANIC CHEMISTRY, AMERICAN CHEMICAL SOCIETY. EASTON, US, vol. 65, 2000, pages 4900-4908, XP000992973 ISSN: 0022-3263

### SEQUENCE LISTING

<110> DR. Chip Biotechnology Incorporation
<120> Diagnostic Assay of Genetic Mutations by Discriminating Amplification and Hybridization Background
<130> 51020
<150> US 09/915,780
   <151> 2001-07-26
<160> 24
<170> Patent In version 3.1
<210> 1
   <211> 28
   <212> DNA
   <213> Artificial Sequence
<220>
   <223> primer
<220>
   <221> misc_feature
   <223> biotin at 5' terminus
<400> 1
   gaattcacca gcataagaga atggggag 28
<210> 2
   <211> 36
   <212> DNA
   <213> Artificial Sequence
<220>
   <223> primer
<400> 2
   agttctagag cgctcgagcc atcaggtagt acagat 36
<210> 3
   <211> 37
   <212> DNA
   <213> Artificial Sequence
<220>
   <223> primer
<400> 3
   gagtcgtatt acggatcctc catcaggtag tacagac 37
<210> 4
   <211> 51
   <212> DNA
   <213> Artificial Sequence
<220>
   <223> oligonucleotide for analysis of amplification products
<400> 4
   tttttttttt tttttttttt tttttctgat ggaggatccg taatacgact c 51
<210> 5
   <211> 50
   <212> DNA
   <213> Artificial Sequence
<220>
   <223> oligonucleotide for analysis of amplification products
<400> 5
   tttttttttt tttttttttt tttttctgat ggctcgagcg ctctagaact 50
<210> 6
   <211> 50
   <212> DNA
   <213> Artificial Sequence
<220>
   <223> oligonucleotide for positive controls
<400> 6
   tttttttttt tttttttttt tttttctccc aaatcctggc aggtcaggca 50
<210> 7
   <211> 50
   <212> DNA
   <213> Artificial Sequence
<220>
   <223> oligonucleotide for positive controls
<400> 7
   tttttttttt tttttttttt tttttggcag gtcaggcagt tgcgttcaac 50
<210> 8
   <211> 43
   <212> DNA
   <213> Artificial Sequence
<220>
   <223> oligonucleotide for negative control
<400> 8
   tttttttttt tttttttttt tttttctagt tattgctcag cgg 43
<210> 9
   <211> 20
   <212> DNA
   <213> Artificial Sequence
<220>
   <223> primer
<220>
   <221> misc_feature
   <223> biotin at 5' terminus
<400> 9
   gcttcatcag tggctcagtg 20
<210> 10
   <211> 38
   <212> DNA
   <213> Artificial Sequence
<220>
   <223> primer
<400> 10
   gagtcgtatt acggatccga ggtgcccagg aggtagaa 38
<210> 11
   <211> 40
   <212> DNA
   <213> Artificial Sequence
<220>
   <223> control probe
<400> 11
   tttttttttt tttttttttt taggatccgt aatacgactc 40
<210> 12
   <211> 412
   <212> PRT
   <213> Bos taurus
<400> 12
   Met Ser Gly Lys Gly Ser Val Val Leu Ala Tyr Ser Gly Gly Leu Asp
   1               5                   10                  15
   Thr Ser Cys Ile Leu Val Trp Leu Lys Glu Gln Gly Tyr Asp Val Ile
               20                  25                  30
   Ala Tyr Leu Ala Asn Ile Gly Gln Lys Glu Asp Phe Glu Glu Ala Arg
           35                  40                  45
   Lys Lys Ala Leu Lys Leu Gly Ala Lys Lys Val Phe Ile Glu Asp Ile
       50                  55                  60
   Ser Lys Glu Phe Val Glu Glu Phe Ile Trp Pro Ala Ile Gln Ser Ser
   65                  70                  75                  80
   Ala Leu Tyr Glu Asp Arg Tyr Leu Leu Gly Thr Ser Leu Ala Arg Pro
                   85                  90                  95
   Cys Ile Ala Arg Lys Gln Val Glu Ile Ala Gln Arg Glu Gly Ala Lys
                   100                 105                 110
   Tyr Val Ser His Gly Ala Thr Gly Lys Gly Asn Asp Gln Ile Arg Phe
           115                 120                 125
   Glu Leu Thr Cys Tyr Ser Leu Ala Pro Gln Ile Lys Val Ile Ala Pro
       130                 135                 140
   Trp Arg Met Pro Glu Phe Tyr Asn Arg Phe Gln Gly Arg Asn Asp Leu
   145                 150                 155                 160
   Met Glu Tyr Ala Lys Gln His Gly Ile Pro Val Pro Val Thr Pro Lys
                   165                 170                 175
   Asn Pro Trp Ser Met Asp Glu Asn Leu Met His Ile Ser Tyr Glu Ala
               180                 185                 190
   Gly Ile Leu Glu Asn Pro Lys Asn Gln Ala Pro Pro Gly Leu Tyr Thr
           195                 200                 205
   Lys Thr Gln Asp Pro Ala Lys Ala Pro Asn Ser Pro Asp Met Leu Glu
       210                 215                 220
   Ile Glu Phe Lys Lys Gly Val Pro Val Lys Val Thr Asn Val Gly Asp
   225                 230                 235                 240
   Gly Thr Thr His Ser Thr Ala Leu Glu Leu Phe Leu Tyr Leu Asn Glu
                   245                 250                 255
   Val Ala Gly Lys His Gly Val Gly Arg Ile Asp Ile Val Glu Asn Arg
               260                 265                 270
   Phe Ile Gly Met Lys Ser Arg Gly Ile Tyr Glu Thr Pro Ala Gly Thr
           275                 280                 285
   Ile Leu Tyr His Ala His Leu Asp Ile Glu Ala Phe Thr Met Asp Arg
       290                 295                 300
   Glu Val Arg Lys Ile Lys Gln Gly Leu Gly Leu Lys Phe Ala Glu Leu
   305                 310                 315                 320
   Val Tyr Thr Gly Phe Trp His Ser Pro Glu Cys Glu Phe Val Arg His
                   325                 330                 335
   Cys Ile Ala Lys Ser Gln Glu Arg Val Glu Gly Lys Val Gln Val Ser
               340                 345                 350
   Val Phe Lys Gly Gln Val Tyr Ile Leu Gly Arg Glu Ser Pro Leu Ser
           355                 360                     365
   Leu Tyr Asn Glu Glu Leu Val Ser Met Asn Val Gln Gly Asp Tyr Glu
       370                 375                 380
   Pro Val Asp Ala Thr Gly Phe Ile Asn Ile Asn Ser Leu Arg Leu Lys
   385                 390                 395                     400
   Glu Tyr His Arg Leu Gln Asn Lys Val Thr Ala Lys
                   405                 410
<210> 13
   <211> 1398
   <212> DNA
   <213> Bos taurus
<400> 13
   cccgtcacga tgtccggcaa aggctccgtg gttctggcct acagtggggg cctggacacc    60
   tcctgcatcc tcgtgtggct gaaggagcaa ggctatgacg tcattgccta cctggccaac   120
   atcggccaga aagaagactt tgaggaagcc aggaagaagg cgctgaagct tggggccaaa   180
   aaggtgttca ttgaggacat cagcaaggag tttgtggagg agttcatctg gccggccatc   240
   cagtccagcg cactgtacga ggaccgatac ctcctgggca cctctctcgc caggccctgc   300
   atcgcccgca agcaggtgga gatcgcccag cgagaaggag ccaagtatgt gtctcacggc   360
   gccacaggaa aggggaacga ccagatccgg tttgagctca cctgctactc gctggcccca   420
   cagatcaagg tcatcgctcc ctggaggatg cccgagttct ataaccgctt ccagggccgc   480
   aacgatctga tggagtatgc gaagcaacat ggaatccccg tcccagtcac ccccaagaac   540
   ccgtggagca tggacgagaa cctgatgcat atcagctacg aggctggaat cctggagaac   600
   cccaagaacc aagcgcctcc aggcctctac acaaagaccc aggacccggc caaagccccc   660
   aacagcccgg acatgctcga gatcgagttc aagaaagggg tccccgtgaa ggtgaccaac   720
   gtcggggatg gcaccaccca cagcacagcg ttggagcttt tcctgtacct gaatgaagtc   780
   gctggcaagc acggcgtggg ccgcatcgac atcgtggaaa accgcttcat cgggatgaag   840
   tcccggggta tctacgagac cccagcgggg acgatccttt accacgctca tttagacatc   900
   gaggccttca ccatggaccg ggaagtgcgc aaaatcaagc aaggcctcgg cttgaaattc   960
   gccgagctgg tctacacggg tttctggcac agccccgagt gtgaatttgt ccgccactgc  1020
   attgccaagt cccaggagcg cgtggaaggg aaagtgcagg tgtccgtctt caagggccag  1080
   gtgtacatcc ttggccggga gtccccactg tccctctaca atgaggagct cgtgagcatg  1140
   aacgtgcagg gagactacga gccggttgat gccactggtt tcatcaacat caattccctc  1200
   aggctgaagg aatatcatcg cctccagaac aaggtcaccg ccaaatagac cagctgacaa  1260
   tgcagagctg gggccgcctc actttaccca tctcccaagt gtagctgcta attgttgtga  1320
   taatttgtga ttgtgacttg ttctctgggt ctggcagcgt agtgggggtg ccaggcctca  1380
   gctttgttcc aatagtcg                                                1398
<210> 14
   <211> 19
   <212> PRT
   <213> Bos taurus
<400> 14
   Arg Lys Lys Ala Leu Lys Leu Gly Ala Lys Lys Val Phe Ile Glu Asp
   1               5                   10                  15
   Ile Ser Lys
<210> 15
   <211> 1367
   <212> DNA
   <213> Bos taurus
<400> 15
   caggaagaag gcgctgaagc ttggggccaa aaaggtacca agtgtgggca gggtttgggg      60
   ctggaaatgg gatctgacac gttggggtca gctgtggggg tccctgtccc agagctcctt     120
   gcctcctacg ccccttccag ttggtcctct tgcttccaag agtgtgaggt caattccttc     180
   cgctgttggt gtgagaactt ctcttatagc tcagctggta aagaatctgc ctgcaatgca     240
   ggagacctgg gttcaattcc tgggttggaa agatcccctg gagaagggaa aggctaccca     300
   ctccagtgtt ctggcctgga gaattccatg gactgtatag tccctggggt cacaaagagt     360
   tggacacgac tgagtgactt tcactttgct gtgtgaacca tcttctagtt tctcctagcc     420
   tctcttgcac ccaatcacct ccccatccca cctccccatc tgtctacctc ttcctcatcc     480
   atccattcac ctgtgcaccc ctcatccatc tacctgtcca ccctcccatc cttccatcca     540
   ctcaacagta tcagtgagca ggccatcaga tgaacatggc cgtcatggtc cctgctcacg     600
   taagctcaca gtacgaagga aacacagtat ctgtgaagtg caataaaact cacatcagaa     660
   gtattaggaa gctgtcaagt tcacagtgtc agatacaagt tttccataat tctagttttt     720
   ttgctggaaa gcttgagttt tatcactggc aacaaatact cagttttcct tgaagtgaca     780
   gggtcacttc atttttatgc aaatcgtggc caaaaaccca agtctgattg atcttgtttt     840
   gcctgtcagt ccttcagtca agtatgaatg atggtttatg agaagagtgg ctaatttagc     900
   tagtaactca cttgggcacg cagcagaaat gctttgtgtg tgtttatcat attttgtatt     960
   caagagttgg ggcttcatca gtggcccagt ggtaaagaat ccaccagcga tgcaggagac    1020
   acaggagaca caggttcaat ccctgggtca ggaagactct ggaggagggc atggcaaccc 1080
   actttaggat tcttgcctgc aaaatcccat ggacagcgga gcctggtggg ctatagtcta 1140
   tagggtcaca gagtcggaca cagccgaagt gacatagcat gcactcaaga atcgagattt 1200
   aacaaaagtg aatcgttttc tactgcattt tcaagaccac cctgttagct gcgtctgcct 1260
   tagacacagg ccatcggaga ctctgtcttc tgacggtgcc ctgtccttgc caaggctccc 1320
   tcccatgggc tgctctccca caggtgttca ttgaggacat cagcaag 1367
<210> 16
   <211> 19
   <212> DNA
   <213> Artificial Sequence
<220>
   <223> oligonucleotide
<400> 16
   caggaagaag gcgctgaag 19
<210> 17
   <211> 18
   <212> DNA
   <213> Artificial Sequence
<220>
   <223> oligonucleotide
<400> 17
   ccgtgagaca catacttg 18
<210> 18
   <211> 20
   <212> DNA
   <213> Artificial Sequence
<220>
   <223> oligonucleotide
<220>
   <221> misc_feature
   <223> biotin at 5' terminus
<400> 18
   gcttcatcag tggctcagtg 20
<210> 19
   <211> 38
   <212> DNA
   <213> Artificial Sequence
<220>
   <223> oligonucleotide
<400> 19
   gagtcgtatt acggatccga ggtgcccagg aggtagaa 38
<210> 20
   <211> 47
   <212> DNA
   <213> Artificial Sequence
<220>
   <223> oligonucleotide
<400> 20
   tttttttttt tttttttttt ttgaaaatgc agtagaaaac gattcac 47
<210> 21
   <211> 47
   <212> DNA
   <213> Artificial Sequence
<220>
   <223> oligonucleotide
<400> 21
   tttttttttt tttttttttt aaactccttg ctgatgtcct caatgaa 47
<210> 22
   <211> 47
   <212> DNA
   <213> Artificial Sequence
<220>
   <223> oligonucleotide
<400> 22
   tttttttttt tttttttttt gaactcctcc acaaactcct tgctgat 47
<210> 23
   <211> 39
   <212> DNA
   <213> Artificial Sequence
<220>
   <223> oligonucleotide
<400> 23
   tttttttttt tttttttttt tggatccgta atacgactc 39
<210> 24
   <211> 54
   <212> DNA
   <213> Artificial Sequence
<220>
   <223> oligonucleotide
<400> 24
   tttttttttt tttttttttt tttttatgaa gcaygtcagg gcrtggatac ctcg 54

## Description

This invention relates a novel primer capable to discriminate between two nucleic acids which differ by only one base, and which may be used to detect a single nucleotide polymorphism. The present invention also pertains to a method for detecting a polymorphism in a nucleic acid.

Single nucleotide polymorphisms, a set of single nucleotide variants at genomic loci, are distributed throughout a genome. In the human genome, such single nucleotide variation occurs relatively frequently, about once in every 200-1000 bases, resulting in millions of single nucleotide polymorphisms (Collins, et al. (1997) Science 278: 1580). In general, when a single nucleotide polymorphism exists at a locus within a gene for a structure protein, the variant may be dominant. On the other hand, when a single nucleotide polymorphism is at a locus within a gene for a catalytic enzyme, the variant may be recessive. See Beaudet et al. (1989) The Metabolic Basis of Inherited Disease 6th Ed. Scriver et al. (Eds) McGraw-Hill Publishing Co. New York, pp 13. In animals, genetic recessive disorders caused by a polymorphism may include bovine leukocyte adhesion deficiency (BLAD, Shuster et al. (1992) Pro. Acad. Natl. Sci. USA 89: 9225-9229), citrullinemia (Dennis et al. (1989) Pro. Acad. Natl. Sci. USA 86: 7947-7951), maple syrup urine disease (MSUD, Zhang et al. (1990) J. Biol. Chem. 265: 2425), deficiency of uridine monophosphate synthase (DUMPS, Shanks et al. (1987) J. Anim. Sci. 64: 695-700), α-mannosidosis (Jolly (1993) Vet. Clin. N. Am. 9: 41), and generalized glycogenosis (Pompes Disease; Dennis et al. (2000) Mamm. Genome 11: 206). In humans, an example of genetic recessive disorders is cystic fibrosis (Kerem et al. (1989) Science 245: 1073-1080), which affects about 1/2000 individuals of the entire Caucasian population.

A single nucleotide polymorphism can be "allelic." That is, due to the existence of the polymorphism, some members of a species may have the unmutated sequence (i.e. the wild-type allele) whereas other members may have a mutated sequence (i.e. the mutant allele). Further, for each polymorphism, there are three possible genotypes: homozygous wild-type alleles, homozygous mutant alleles, and heterozygous alleles.

The problem of the present invention is to provide an efficient method for detecting a single nucleotide polymorphism, including genotyping.

This problem has been solved by providing a novel primer that discriminates between two nucleic acids which differ by only one base, and therefore, can be used to detect a single nucleotide polymorphism.

More specifically, one aspect of this invention features a discrimination primer for amplifying a nucleic acid that includes a first base at a position suspected of a polymorphism and a second base immediately 3' to the first base. This primer includes (1) a first nucleotide, which is located at the 3' terminus of the primer and contains a base that is complementary to the first base; (2) a second nucleotide, which is located immediately 5' to the first nucleotide and contains a base that is not complementary to the second base; (3) a segment of nucleotides (e.g., 5 to 50, or 10 to 40 nucleotides in length), which is located immediately 5' to the second nucleotide and is complementary to a part of the nucleic acid that is immediately 3' to the second base; and (4) a binding member of a specific binding pair covalently bonded to the 5' terminus of the segment. The first base of the nucleic acid can be mutant or wild-type.

A nucleic acid targeted to be amplified can be DNA (ss or ds DNA) or RNA, in a purified or unpurified form. It also can be a genomic fragment or a restriction fragment. The term "complementary" refers to a sequence forming a duplex with another sequence when these sequences base pair with one another, perfectly or partly. In a perfect duplex, two sequences are precisely complementary. In a partial duplex, two sequences have at least one, two, or more mismatched base pairs, but are still capable of synthesizing a primer extension product. A specific binding pair refers to two binding members that specifically bind to one another. It can be a protein-ligand pair (e.g., streptavidin-biotin), a hybridizing nucleic acid pair, a protein-protein pair, an antibody-antigen pair, or a nucleic acid-nucleic acid binding protein pair. For example, a binding member of the specific binding pair is an oligonucleotide that is not complementary to any part of the nucleic acid to be amplified, and the other member of the specific binding pair is also an oligonucleotide; both binding members can be 6 to 50 nucleotides (e.g., 10 to 40 nucleotides) in length. One binding member forms an integral part of the discrimination primer, and thus also of an amplification product extended therefrom. Via the binding member, the amplification product binds to the other binding member, which is immobilized (directly or indirectly) on a solid substrate. Affixation of the amplification product to a solid substrate facilitates its detection.

In another aspect, this invention features a method for detecting a polymorphism in a nucleic acid. The method includes (1) providing a nucleic acid containing a base at a position suspected of a polymorphism; (2) amplifying the nucleic acid with a first binding member-containing discrimination primer (as described above) and another primer (an amplification primer); (3) contacting the amplified nucleic acid with a second binding member capable of binding to the first binding member; and (4) detecting the amplified nucleic acid that binds to the second binding member. Optionally, this method includes amplifying the nucleic acid in the presence of two discrimination primers, one of which includes a mutant base in the nucleic acid sequence, and the other primer includes a wild-type base. The term "amplifying" as used herein refers to the process of producing multiple copies of a desired sequence of the provided nucleic acid or a portion thereof, e.g., 50 to 1000 nucleotides in length.

An amplification primer, together with a discrimination primer, is used to amplify a nucleic acid including a polymorphism. It can include a label at its 5' terminus. The label can be detected, directly or indirectly, by well-known techniques. Examples of the label include, but are not limited to, a fluorescent molecule (e.g., fluorescein and rhodamine), biotin (which can be detected by an anti-biotin specific antibody or an enzyme-conjugated avidin derivative), a radioactive isotope (e.g., ³²P or ¹²⁵I), a calorimetric reagent, and a chemiluminescent reagent.

Also within the scope of this invention is a kit for detecting a polymorphism. The kit includes a discrimination primer and an amplification primer as described above. Optionally, the kit can include two discrimination primers, one containing a mutant base at its 3' terminus, and the other containing a wild-type base at its 3' terminus. When only one discrimination primer is included, the kit can be used to analyze a polymorphism. When two discrimination primers are included, the kit can be used to further determine the genotype of polymorphic alleles. In addition to the primers, the kit may further include an enzyme (i.e., DNA polymerase) and reagents for amplification (e.g., nucleotides, or analogs thereof such as deoxyinosine). It can also include solid substrates, such as glass plates or plastic microchips containing arrays of oligonucleotides (i.e., various binding members to be used to bind amplification products).

Other features, objects, and advantages of the invention will be apparent from the description and from the claims.

The present invention relates to a discrimination primer, and an amplification primer, for use in an amplification reaction to detect a polymorphism.

A "primer" is an oligonucleotide capable of acting as a point of initiation of synthesis of a primer extension product that is complementary to a nucleic acid strand (template or target sequence), when placed under suitable conditions (e.g., salt concentration, temperature, and pH) in the presence of nucleotides and other reagents for nucleic acid polymerization (e.g., a DNA dependent polymerase). As known in the art, a primer must be of a sufficient length (e.g., at least 6 nucleotides) to prime the synthesis of extension products.

Use of a discrimination primer and an amplification primer allows for preferential (e.g., exclusive) amplification of a nucleic acid that contains a polymorphism. In other words, such a primer pair can be used to preferentially amplify one polymorphic allele (e.g., mutant allele) over the other (e.g., wild-type allele). The discrimination primer includes a first nucleotide, which is located at the 3' terminus of the primer and contains a base that is complementary to a first base suspected of a polymorphism in a nucleic acid; a second nucleotide, which is located immediately 5' to the first nucleotide and contains a base that is not complementary to a second base immediately 3' to the first base; a segment of nucleotides; and a binding member of a specific binding pair. The oligonucleotide consisting of the first and second nucleotides and the segment (at least 5 bases in length) is capable of acting as a point of initiation of synthesis of a primer extension product. When the first base in the nucleic acid is wild-type, a discrimination primer containing a mutant nucleotide at its 3' terminus has one more mismatched base when annealing to a wild-type allele than annealing to a mutant allele. More specifically, this discrimination primer has two mismatched bases at its 3' end when annealing to a wild-type allele, and is therefore not able to act as a point of initiation of synthesis of a primer extension reaction. Conversely, when the first base is mutant, the just-described discrimination primer has only one mismatched base at its 3' end, and can be used to act as a point of initiation of synthesis of a primer extension reaction.

A discrimination primer can be optimized on a gene-by-gene basis to provide the greatest degree of discrimination between amplification of the wild-type allele and the mutant allele. The optimization will of necessity include some empirical observations, but a number of basic principles can be applied to select a suitable starting point for final optimization. A discrimination primer can be designed based on a known single nucleotide polymorphism in a gene, and also based on its properties, e.g., GC-content, annealing temperature, or internal pairing, which can be analyzed using software programs. As discussed above, a discrimination primer of this invention includes a first binding member, such that an amplification product can bind, via the first binding member, to a second binding member immobilized on a solid substrate. If both binding members are oligonucleotides, the optimization may further take account of annealing or other properties of the first and second binding members. Of course, one must confirm empirically the ability of a discrimination primer to amplify a mutant allele or a wild-type allele.

A primer pair of this invention can be used to selectively amplify a nucleic acid with a single nucleotide polymorphism. The nucleic acid can be obtained from any suitable source, e.g., a tissue homogenate, blood, amniotic fluid, or chorionic villus samples; and can be DNA or RNA (in the case of RNA, reverse transcription is required before PCR amplification). PCR amplification can be carried out following standard procedures. See, e.g., Ausubel et al. (1989) Current Protocols in Molecular Biology John Wiley and Sons, New York; Innis et al. (1990) PCR Protocols: A Guide to Methods and Applications Academic Press, Harcourt Brace Javanovich, New York. More specifically, a method of discriminating amplification has been described in, for example, Cha *et al.* (1992) *PCR Methods and Applications* 2: 14. Unexpectedly, the discrimination primer of this invention, despite the presence of a first binding member at its 5' terminus, can still efficiently produce a specific amplification product. A discrimination primer that contains an oligonucleotide as the first binding member can be prepared by a synthetic method, or alternatively, by a ligation method (e.g., a method of using cyanogen bromide described in Selvasekaran and Turnbull (1999) Nucleic Acids Res. 27(2): 624). A discrimination primer that contains a peptide as the first binding member can be prepared by conjugation of a peptide and an oligonucleotide based on, e.g., a "native ligation" of an N-terminal thioester-functionalized peptide to a 5'-cysteinyl oligonucleotide. See Stetsenko and Gait (2000) J. Org. Chem. 65(16): 4900.

Detection of an amplification product of a polymorphism-containing nucleic acid indicates the presence of a wild-type or mutant allele. According to the method of this invention, an amplification product is detected on a solid substrate when a first binding member, an integral part of the amplification product, binds to a second binding member that is immobilized on a solid substrate. Affixing the amplification product to a solid substrate facilitates its detection. The second binding member can be directly immobilized on a solid support. It also can be indirectly immobilized on a solid substrate. More specifically, if the second binding member has a segment binds to the first binding member and has another segment that binds to a third binding member that has been immobilized on a solid substrate, it can be immobilized on the solid substrate via binding to the third binding member.

One can immobilize a second binding member on a solid substrate by attaching it to the substrate via a covalent or non-covalent bonding. Alternatively, a second binding member can be formed on the substrate by attaching a precursor molecule to the substrate and subsequently converting the precursor to the second binding member, such as *de novo* synthesis of nucleic acid at a precise region on the solid substrate by a photolithographic method. For example, see, Schena et al. (1995) Science 270: 467; Kozal et al. (1996) Nature Medicine 2(7): 753; Cheng et al. (1996) Nucleic Acids Res. 24(2): 380; Lipshutz et al. (1995) BioTechniques 19(3): 442; Pease et al. (1994) Proc. Natl. Acad. Sci. USA 91: 5022; Fodor et al. (1993) Nature 364: 555-; and Fodor et al., WO 92/10092. The solid substrate can be an agarose, acrylamide, or polystyrene bead; a nylon or nitrocellulose membrane (for use in, e.g., dot or slot blot assays); a glass or plastic polymer; a silicon or silicon-glass (e.g., a microchip); or gold (e.g., gold plates).

An amplification product is detected after it binds to an immobilized second binding member. To enable detection, the amplification product can be labeled by using a labeled amplification primer, or can be labeled, chemically or enzymatically, after amplification. When only the amplification product or the second binding member is labeled with a fluorescent molecule, the presence of the amplification product can be detected by fluorescence. When both the amplification product and the second binding member are labeled with fluorophores, the amplification product can be detected by monitoring a color shift due to proximity of the fluorophores resulting from binding of the amplification product to the second binding member. Examples of fluorescent labels include, but are not limited to, fluorescein, rhodamines, infrared dyes (e.g., IR-132 or IR-144; Kodak, Rochester, N.Y), and cyanine dyes (e.g., Cy3 or Cy5; Amersham Int'l, Cleveland). See Ranki et al. (1983) Gene 21: 77: Keller et al. (1991) J. Clin. Microbiol. 29: 638; and Urdea et al. (1987) Gene 61: 253.

To determine a genotype at a locus of a polymorphism, an assay can be performed as follows. Two discrimination primers and one amplification primer are used in PCR amplification. One discrimination primer has the 3' terminal nucleotide complementary to a mutant base, and is for use to preferentially amplify a mutant allele. The other discrimination primer has the 3' terminal nucleotide complementary to a wild-type base, and is for use to preferentially amplify a wild-type allele. The first binding members of the two discrimination primers are different oligonucleotides, and bind to different binding partners (i.e., second binding members), which are separately immobilized. Binding of an amplification product to one immobilized binding partner, to the other immobilized binding partner, or to both indicates one of the three possible genotypes. Unexpectedly, this assay has a high sensitivity, i.e., up to 100 folds that an assay in which amplification products are detected on agarose gel.

Use of spatially arrayed binding partners for simultaneously detecting a multiplicity of polymorphisms is within the scope of this invention. See, for example, U.S. Patent Nos. 5,424,186, 5,510,270, and 5,744,305.

The specific example below is to be construed as merely illustrative, and not limitative of the remainder of the disclosure in any way whatsoever. Without further elaboration, it is believed that one skilled in the art can, based on the description herein, utilize the present invention to its fullest extent.

The following examples illustrate the invention without limiting it thereto.

### Example 1: Detection of a single nucleotide polymorphism using plasmid DNA

*Construction of standard BLAD gene fragments.* A cattle recessive genetic disorder, bovine leukocyte adhesion deficiency (BLAD), is caused by a single nucleotide polymorphism (Shuster et al. (1992) Proc. Natl. Acad. Sci. U.S.A. 89: 9225-9229). The study was based on the published gene sequence BTCD18, partial sequence of *B. taurus* CD18 gene (NCBI, Accession No. Y 12672). The single point mutation is located at nucleotide position 1200 of BTCD 18 gene.

A 1341 bp fragment was amplified from the BTCD18 gene, incorporated in a pGEM-T Easy vector system (Promega, Madison, WI, USA), and transformed in a bacterium host, *Escherichia coli.* Nucleic acids were prepared from all transformants and analyzed using the restriction enzyme Taql. The presence or absence of a TaqI-restriction site in a nucleic acid indicates the type of an allele. A wild-type allele possesses the TaqI restriction site, while a mutant allele does not. Two transformants with plasmids pGEM7-BD and pGEM8-BD were selected as representative standard gene fragments for wild-type allele and mutant allele, respectively. Their sequences were confirmed by sequence analysis.

*Discriminating amplification of two genetic alleles*. Discriminating amplification was performed by employing one amplification primer as a forward primer and two discrimination primers as reverse primers. HjT-F8a was designed as an amplification primer. It was 23 nucleotides in length, corresponding to 1020-1042 of the BTCD18 gene sequence: 5'-GAATTCACCAGCATAAGAGAATGGGGAG -3' (SEQ ID NO:1), and had biotin at its 5'-terminus. Two discrimination primers were R11-1-3mis18 (wild-type allele specific reverse primer), 5'-AGTTCTAGAGCGCTCGAGCCATCAGGTAGTACAGAT-3' (SEQ ID NO:2), and RM11-1-3mis18 (mutant allele specific reverse primer), 5'-GAGTCGTATTACGGATCCTCCATCAGGTAGTACAGAC-3' (SEQ ID NO:3). The two primers were based on 1218-1200 and 1217-1200 of the BTCD18 gene sequence, respectively, and their first binding members are underlined. The ultimate 3' base of wild-type (mutant) specific reverse primer was designed as a complementary base to the wild-type (mutant) allele. Each reverse primer had one mismatched base at penultimate position of the 3'-terminus. All primers were commercially prepared by standard oligonucleotide synthesis techniques (e.g., GENESET Singapore Biotech. Pte Ltd, Singapore).

An amplification reaction was performed as follows. The reaction volume was 50 µL and the reaction mixture contained 50-100 ng DNA template, 5 µL 10X Taq DNA polymerase buffer, 5 µL 15 mM MgCl₂, 250 µM dNTP each (Promega), 400 nM primer each, 2-2.5 units Taq DNA polymerase (Promega), and dH₂O. A two-step amplification reaction was employed with temperature parameters of 94°C for 30 sec and 55°C for 20 sec for 30-35 cycles after one cycle with 94°C for 4 min. Amplification conditions were carried out using RoboCycler Temperature Cycler (Strategene).

The amplification products were analyzed by using agarose gel electrophoresis. The gel showed that a specific amplification product with the right size (222 bp) was produced when the primers HjT-F8a and R11-1-3mis18 were mixed with the template pGEM7-BD (the wild-type allele), but not with the template pGEM8-BD (the mutant allele). Similarly, the primers HjT-F8a and RM11-1-3mis18 were able to amplify the mutant allele pGEM8-BD, but were not able to amplify the wild-type allele pGEM7-BD.

*Analysis of the amplification products by hybridization.* Three types of the second binding members (i.e., oligonucleotides) were designed for analysis of amplification products of the mutant allele and the wild-type allele. Type 1 oligonucleotides were designed to identify the amplification products, i.e., HjT-P1, 5'-(T)₂₅-CTGATGGAGGATCCGTAATACGACTC-3' (SEQ ID NO:4), and HjT-PM1, 5'-(T)₂₅-CTGATGGCTCGAGCGCTCTAGAACT-3' (SEQ ID NO:5). The two underlined sequences are complementary to the first binding members of RM11-1-3mis18 (19 bp) and R11-1-3mis18 (17 bp), respectively. Type 2 oligonucleotides were designed for positive controls, i.e., HjT-Pco3, 5'-(T)₂₅-CTCCCAAATCCTGGCAGGTCAGGCA-3' (SEQ ID NO:6) and HjT-Pco4, 5'-(T)₂₅-GGCAGGTCAGGCAGTTGCGTTCAAC-3' (SEQ ID NO:7). Both sequences correspond to two regions of BTCD18 gene (1129-1153 and 1141-1165), respectively). A type 3 oligonucleotide was designed for a negative control, i.e., HjT-Nco1, 5'-(T)₂₅-CTAGTTATTGCTCAGCGG-3' (SEQ ID NO:8), not homologous to any BTCD18 gene sequence.

The oligonucleotide was dissolved in a probe solution (DR. Probsol, DR.Chip Biotechnology Inc., Taiwan) with a final concentration of 10 µM, spotted, and immobilized on a solid substrate.

The amplification product from each discriminating amplification reaction mixture was diluted with a hybridization buffer in a ratio of 1 : (50-100). The diluted fraction was boiled for 5 min, chilled on ice, and applied to the just described solid support. The hybridization reaction was performed at 50-55°C for 1-2 hours using an oven. Then the solid support was washed with a wash buffer (0.5 mL) (DR. Wash, DR.Chip Biotechnology Inc., Taiwan) for at least three times. Biotin-specific colorimetric detection was performed by incubating the solid substrate with a Blocking Reagent (Roche), which contained alkaline phosphatase-conjugated streptavidin (Promega). Subsequently, the solid substrate was washed three times with the wash buffer, and incubated with NBT/BCIP solution (Roche), which was diluted with a detection buffer in a ratio recommended by the supplier for about 10 min in the dark. The results show that each discriminating amplification product was specifically recognized by its corresponding type 1 oligonucleotide, and all amplification products were recognized by each type 2 oligonucleotide. Detection of colored spots on the positions of HjT-P1 indicated the presence of an amplification product of a mutant allele from the primers HjT-F8a and RM11-1-3mis18. An amplification product of a wild-type allele from the primers HjT-F8a and R11-1-3mis18 was detected as colored spots on the positions of HjT-PM1. Unexpectedly, the detection on the solid substrate was about 10-100 times more sensitive than analysis on ethidium bromide-stained agarose gel.

### Example 2: Detection of a single nucleotide polymorphism using genomic DAN isolated from blood and milk samples

*Genomic DNA isolated from blood samples.* Two whole blood samples, one from a healthy cow and the other from a BLAD carrier, were obtained from Hsinchu Branch, Taiwan Livestock Research Institute (Council of Agriculture, Executive Yuan). Each whole blood sample was transferred to a tube containing EDTA (1-2 mg/mL) to avoid clotting. To prepare the genomic DNA, the whole blood sample was centrifuged at 3,000 xg for 5 min. After centrifugation, three layers were distinguishable: the upper layer was plasma, the intermediate layer contained concentrated leukocytes, and the bottom layer contained concentrated erythrocytes. The genomic DNA was extracted from the intermediate layer using QIAmp Blood Kit (QIAGEN, Hilden, Germany). About 10-20 µg genomic DNA was obtained from 1 mL whole blood sample.

*Genomic DNA isolated from milk samples.* Two milk samples, one from a healthy cow and the other from a BLAD carrier, were obtained from a local farm. Genomic DNA was extracted from a 15 mL milk sample by the alkalic lysis method as described in Shuster et al. (1992) Proc. Acad. Natl. Sci. U.S.A. 89: 9225-9229. The genomic DNA in the aqueous lysate was further purified by adding an organic mixture (phenol/chloroform (1:1)), followed by centrifugation at the maximum speed for 10 min at 4°C. After centrifugation, the aqueous layer was transferred to a tube. The genomic DNA was precipitated with 95% of ice-cold ethanol.

*Discrimination amplification*. All primers and probes were the same as those described in Example 1. All reagents for amplification reactions were also the same, except two microliters of genomic DNA isolated from blood and milk samples were used for amplification. A three-step amplification reaction was employed with temperature parameters of 95°C for 4 min; 10 cycles of 95°C for 60 sec; 52°C for 60 sec; 72°C for 60 sec; 25 cycles of 95°C for 30 sec; 60°C for 30 sec; 72°C for 30 sec; and 72°C for 5 min. The amplification products were analyzed by using the agarose gel electrophoresis and the hybridization method as also described in Example 1. An amplification product of a wild-type allele was detected in the samples isolated from the healthy cow, and amplification products of wild-type and mutant alleles were detected in the samples isolated from the BLAD carrier.

### Example 3: Detection of a single nucleotide polymorphism resulting a cattle recessive genetic disorder, bovine citrullinemia

Bovine citrullinemia is one well-known cattle recessive genetic disorder as it has been mentioned in "Background" section of this patent application. This genetic disorder is an inborn error of urea cycle metabolism and characterized by consequent life-threatening hyperammonemia. The primary cause of bovine citrullinemia is a nonsense mutation, a single nucleotide polymorphism at position 256 (CGA→ TGA), in the bovine argininosuccinate synthase (*B*. *taurus* ASS gene; Accession No. M26198, NCBI GenBank). The single base substitution results in conversion of arginine-86 to a translation termination codon (Dennis *et al*., 1989) and fail in enzyme synthesis.

Detection of bovine argininosuccinate synthase deficiency is currently based on PCR/Restriction and gel electrophoresis method (Dennis et al., 1989). A 176bp-fragment was amplified using a pair of primer, digested by restriction enzyme *Ava*II, and analyzed by gel electrophoresis. The electrophoresis has to be performed either in 4% agarose or 12% acrylamide gel. The amplification of normal wild type allele will expectedly result in production of two small fragments with the molecular size of 79bp and 97bp after gel electrophoresis and the followed ethidium bromide staining, while one 176bp fragment will appear as mutant allele as DNA template. The disadvantage of this current method is that the sizes of these three fragments are quite close and might not be easily distinguished using agarose gel analysis.

We demonstrate a further example for the application of our inventive method for detection of a single nucleotide polymorphism of bovine ASS gene.

### Method and Material

### 1. Construction of standard ASS gene fragments

Only cDNA sequence of bovine ASS gene (Accession No. M26198, NCBI GenBank) was available from NCBI GenBank before our study. The DNA sequence of 176bp, which is applied for current PCR/Restriction-based method, locates in one exon region of bovine ASS gene. More DNA sequence surrounding this exon region, i.e. intron sequence before or after, was needed for designing of the components for discriminating assay.

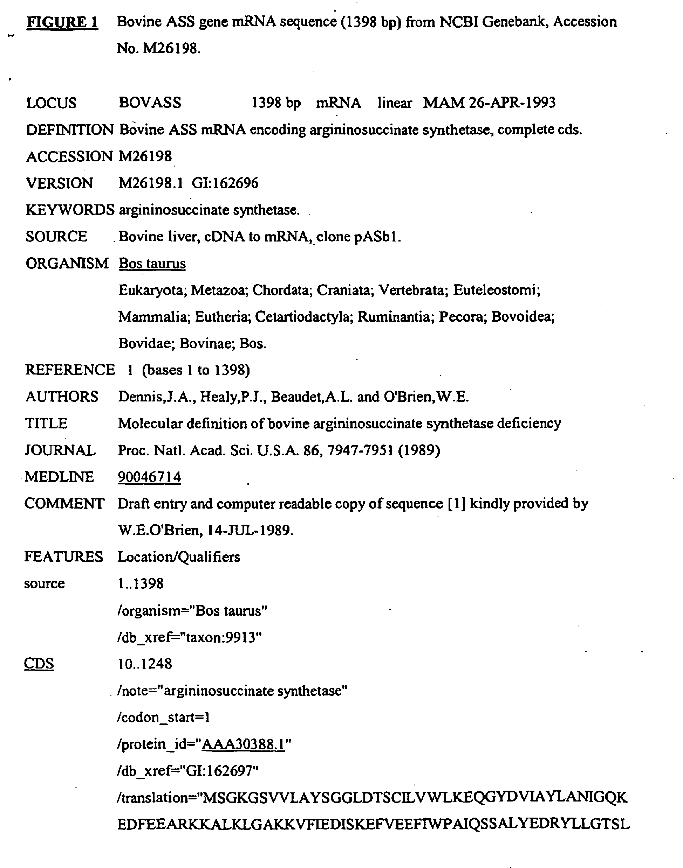

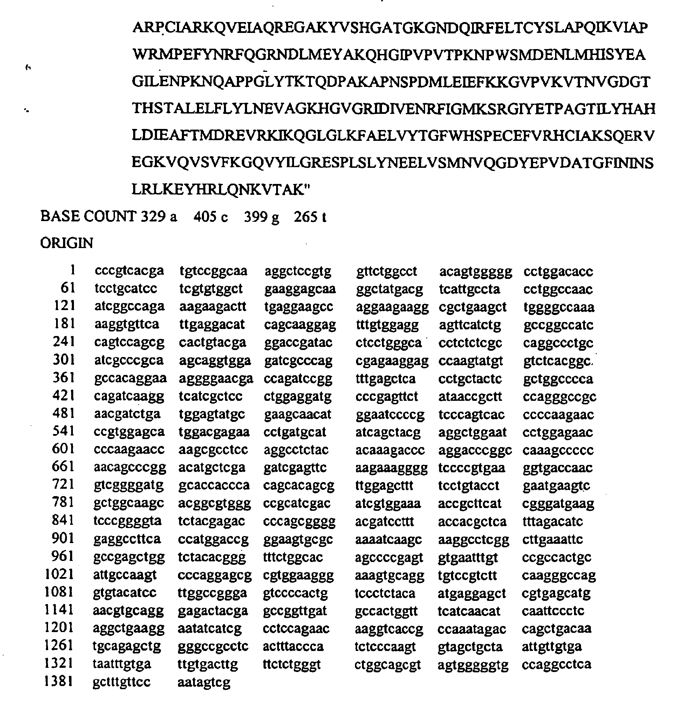

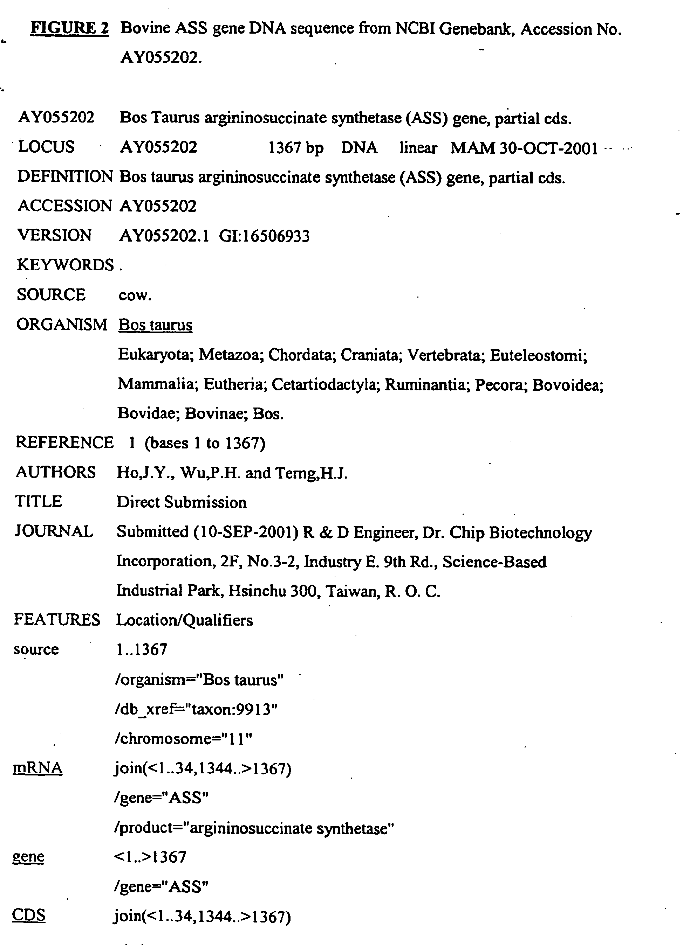

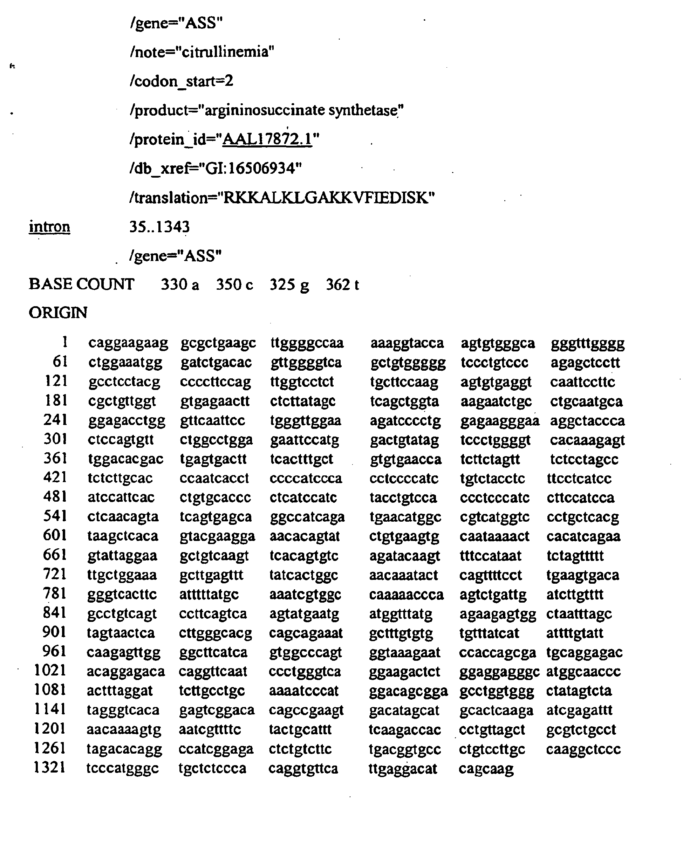

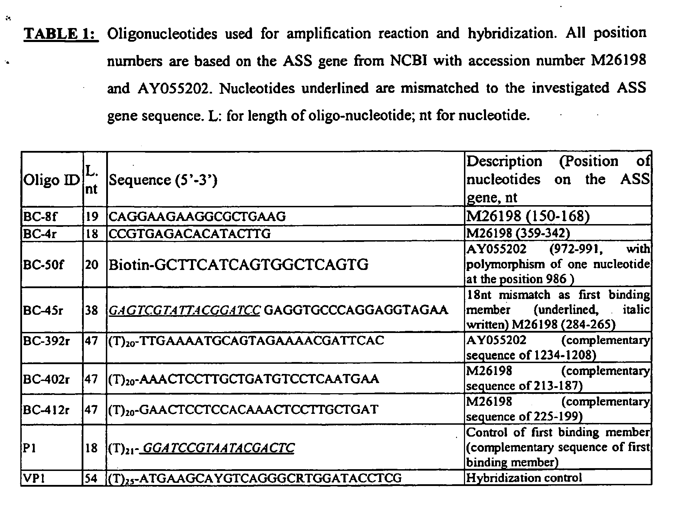

A 1519 bp fragment was amplified from the cattle total genomic DNA with two primers, BC-8f and BC-4r **(TABLE 1).** This partial ASS gene sequence has been accepted by NCBI GenBank with the accession no. AY055202 **(****FIGURE 2****).** The amplified gene fragment was then incorporated in a pGEM-T Easy vector system (Promega, Madison, WI, USA) and transformed in a bacterium host, *Escherichia coli.* Two plasmids pGEM-ASSw and pGEM-ASSm mass-produced from *E. coli* transformants were selected as representative standard gene fragments for wild type allele and mutant type allele, respectively. Their insert sequences are confirmed by sequence analysis.

### 2. Discriminating amplification of two genetic alleles with one different nucleotide

This detection method is aimed at specific identification of the presence of ASS-mutant allele with one different nucleotide of cattle ASS gene, in comparison with normal wild type ASS gene (M21698, NCBI GenBank). The diagnostic finding of the presence and also the frequency of ASS-mutant allele becomes instructive and useful information for cattle genetic breeding, then the identification of wild type-allele. This further example will only focus on construction of optimal discriminating primers, amplification conditions, probes and hybridization system for identification of ASS-mutant allele.

The discriminating amplification could be achieved by employing one primer amplification primer (BC-50f) as forward primer and one discriminating primer as reverse primer (BC-45r). The sequences of inventive primers are listed in **TABLE 1.** BC-50f was designed as an amplification primer. It was 20 nucleotides in length, corresponding to 972-991 of the ASS gene sequence (AY055202): 5'-GCTTCATCAGTGGCTCAGTG with polymorphism of one nucleotide at the position 986, and had biotin at its 5'-terminus.

The discriminating primer was BC-45r, 5'-GAGTCGTATTACGGATCC GAGGTGCCCAGGAGGTAGAA and mutant allele-specific reverse primer. It is based on 284-265 of ASS gene sequence (M26198) and the first binding member is underlined. The ultimate 3' base of mutant specific reverse primer was designed as a complementary base to the mutant allele. The reverse primer had two mismatched bases at penultimate position and the last three postion of the 3'-terminus. All primers were commercially prepared by standard oligo-nucleotide synthesis techniques (e.g. GENESET Singapore Biotech. Pte Ltd, Singapore).

An amplification reaction was performed as follows. The amplification reaction volume is 50µl and the reaction mixture contained 50-100 ng DNA template, 5 µL 10X Taq DNA polymerase buffer, 5 µL 15 mM MgCl₂, 250 µM dNTP each (Promega), 400 nM primer each, 2-2.5 units Taq DNA polymerase (Promega), and dH₂O. A two-step amplification strategy was employed with temperature parameters of 94°C for 1 min and 60°C for 1.5 min for 30-35 cycles after one cycle with 94°C for 4 min. Amplification conditions were carried out using RoboCycler Temperature Cycler (Strategene).

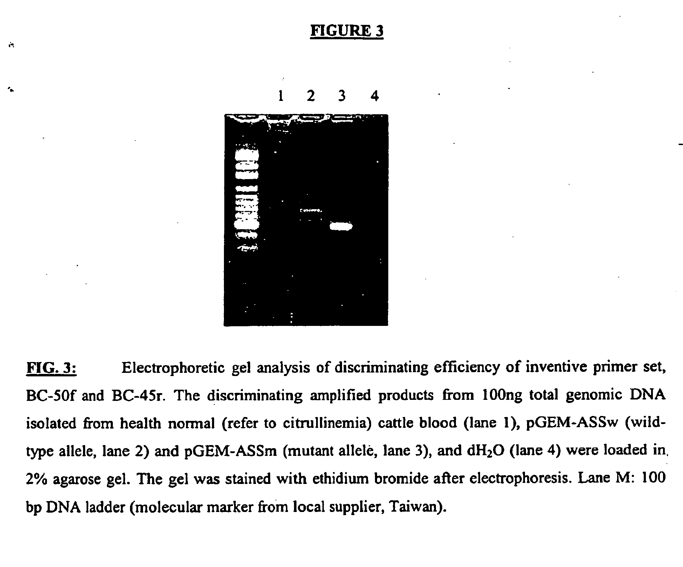

The amplification products were analyzed by using agarose gel electrophoresis. The gel showed that a specific amplification product with the right size (506 bp) was produced when the primers BC-50f and BC-45r with the template pGEM-ASSm (the mutant allele, lane 3, **FIG. 3****),** but not with the template pGEM-ASSw (the wild type allele, lane 2, **FIG. 3****).** There were some unspecific amplified products shown on gel, but they were not the expected product size. The amplification conditions could achieve the discrimination of mutant allele from wild type allele.

### 3. Analysis of the amplification products by hybridization

The amplified products could be sometimes unspecific or unexpected even the molecular size on electrophoresis gel seems to be correct. A following hybridization technique with sequence-specific probes or sequence analysis could be applied for confirmation of the right amplified product. Sequence analysis of DNA fragment does not belong to routine method due to the lack of facility. The hybridization technique could be easily carried out in usual laboratory for molecular biology.

*Probe design.* Three types of probes were designed for analysis of amplification products. Their sequences are shown in the **TABLE 1.**
- Type 1:: Control probe for the presence of the first binding member, i.e. P1, 5'-(T)₂₁-*AGGATCCGTAATACGACTC.* The underlined sequence is complementary to the first binding member of BC-45r.
- Type 2:: Probes for identification of amplified products were designed are BC-392r, BC-402r and BC-412r.
- Type 3:: Probe for hybridization control is VP1, which is complementary to the fragment added extra for controlling of the hybridization reaction.

The oligo-nucleotide was dissolved in a probe solution (DR. Probsol, DR.Chip Biotechnology Inc., Taiwan) with a final concentration of 10 µM, spotted, and immobilized on a solid support.

The amplification product from each discriminating amplification reaction mixture was diluted with a hybridization buffer in a ratio of 1 : (50-100). The diluted fraction was boiled for 5 min, chilled on ice, and applied to the just described solid support. The hybridization reaction was performed at 50-55°C for 1-2 hours using an oven. Then the solid support was washed with a wash buffer (0.5 mL) (DR. Wash, DR.Chip Biotechnology Inc., Taiwan) for at least three times. Biotin-specific colorimetric detection was performed by incubating the solid substrate with a Blocking Reagent (Roche), which contained alkaline phosphatase-conjugated streptavidin (Promega). Subsequently, the solid substrate was washed three times with the wash buffer, and incubated with NBT/BCIP solution (Roche), which was diluted with a detection buffer in a ratio recommended by the supplier for about 10 min in the dark.

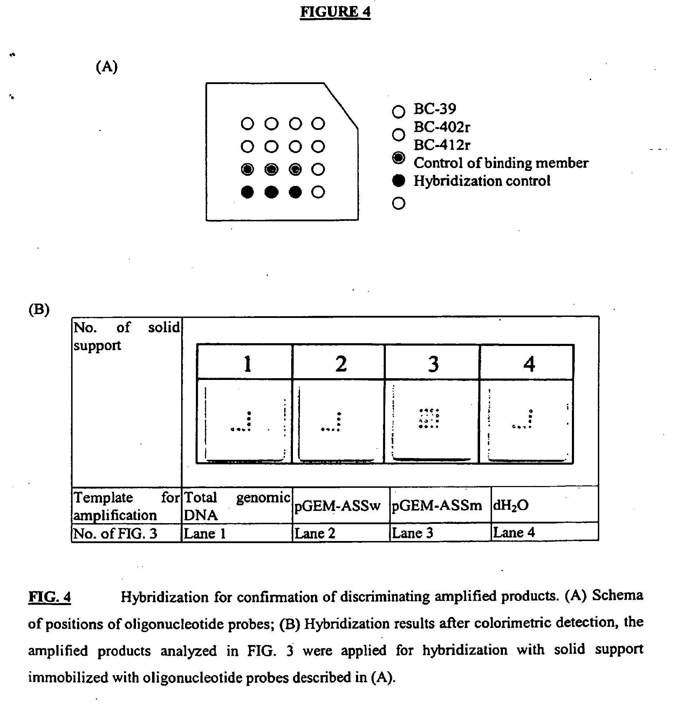

The results show that each discriminating amplification product was specifically recognized by its corresponding type 1 oligonucleotide, and by each type 2 oligonucleotide (No. 3 **FIGURE 4****).** Detection of colored spots on the positions of P1, BC39, BC-402r and BC412r indicated the presence of an amplification product of a mutant allele from the primers BC-50f and BC-45r. The coloring development of solid support no. 1 and no. 4 gave signals only on the position of P1, but not on the positions of BC39, BC-402r and BC412r. These results indicate that the multiple bends derived from amplified products on stained agarose gel were unspecific, since they were not bound by our type 2 oligo-nucleotides. All amplified products performed by using these primers will give the signal on the position of P1, because the reverse primer BC-45r was labeled with first binding member at 5'-terminus. Unexpectedly, the detection on the solid substrate was about 10-100 times more sensitive than analysis on ethidium bromide-stained agarose gel.

All of the features disclosed in this specification may be combined in any combination. Each feature disclosed in this specification may be replace by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the spirit and scope thereof, can make various changes and modifications of the invention to adapt it to various usages and conditions. For example, one can change the number and the position of the mismatched base in a discrimination primer to achieve discriminating amplification.

## Claims

1. A method for amplifying a target nucleic acid that includes a first base at a position suspected of a polymorphism and a second base immediately 3' to the first base comprising the use of a discrimination primer, the primer comprising:
a first nucleotide, which is located at the 3' terminus of the primer and contains a base that is complementary to the first base of the target nucleic acid;
a second nucleotide, which is located immediately 5' to the first nucleotide and contains a base that is not complementary to the second base of the target nucleic acid;
a segment of nucleotides, which is located immediately 5' to the second nucleotide and is complementary to a part of the target nucleic acid that is immediately 3' to the second base; and
a binding member of a specific binding pair covalently bonded to the 5' terminus of the segment.

2. The method of claim 1, wherein the segment of the primer is 5 to 50 nucleotides, especially 10 to 40 nucleotides, in length.

3. The method of claim 2, wherein the binding member of the primer is an oligonucleotide 6 to 50 nucleotides, especially 10 to 40 nucleotides, in length and not complementary to any part of the target nucleic acid.

4. The method of claims 2 or 3, wherein the binding member of the primer is a peptide.

5. A method for detecting a polymorphism in a target nucleic acid, comprising:
providing a target nucleic acid including a first base at a position suspected of a polymorphism and a second base immediately 3' to the first base;
amplifying the target nucleic acid with a first primer and a second primer, wherein the first primer includes a first nucleotide, which is located at the 3' terminus and contains a base that is complementary to the first base of the target nucleic acid, a second nucleotide, which is located immediately 5' to the first nucleotide and contains a base that is not complementary to the second base of the target nucleic acid; a segment of nucleotides, which is located immediately 5' to the second nucleotide and is complementary to a part of the target nucleic acid that is immediately 3' to the second base; and a first binding member of a specific binding pair covalently bonded to the 5' terminus of the segment;
contacting the amplified target nucleic acid with a second binding member of the specific binding pair; and
detecting the amplified target nucleic acid that binds to the second binding member.

6. The method of claim 5, further comprising amplifying the target nucleic acid in the presence of a second first primer, wherein the first nucleotide in one of the two first primers is mutant, and the first nucleotide in the other is wild-type.

7. The method of claims 5 or 6, wherein the second binding member is immobilized on a solid support.

8. The method of claims 5 to 7, wherein the second primer contains a label at the 5' terminus.

9. The method of claims 5, 6 or 7, wherein each of the first binding members of the first primers is, independently, a peptide or an oligonucleotide not complementary to any part of the target nucleic acid.

10. The method of claims 5, 6, 7 or 9, wherein the first binding members of the first primers are different oligonucleotides.

11. The method of claims 5 or 10, wherein the first binding member is an oligonucleotide not complementary to any part of the target nucleic acid.

12. The method of claims 5 or 10, wherein the first binding member is a peptide.

13. Use of a kit in a method according to any of the preceding claims for amplifying a target nucleic acid.

## Patentansprüche

1. Verfahren zum Vermehren einer Ziel-Nukleinsäure, die eine erste Base an einer Position, die vermutlich einen Polymorphismus aufweist, und eine zweite Base unmittelbar 3' zu der ersten Base einschließt, das die Verwendung eines Unterscheidungs-Primers umfasst, wobei der Primer umfasst:
ein erstes Nukleotid, das an dem 3'-Terminus des Primers vorliegt und eine Base beinhaltet, die komplementär zu der ersten Base der Ziel-Nukleinsäure ist,
ein zweites Nukleotid, das unmittelbar 5' zu dem ersten Nukleotid vorliegt und eine Base beinhaltet, die nicht komplementär zu der zweiten Base der Ziel-Nukleinsäure ist,
ein Segment von Nukleotiden, das unmittelbar 5' zu dem zweiten Nukleotid vorliegt und zu einem Teil der Ziel-Nukleinsäure komplementär ist, die unmittelbar 3' zu der zweiten Base ist, und
ein Bindungselement eines spezifischen Bindungspaares, das an den 5'-Terminus des Segments kovalent gebunden vorliegt.

2. Verfahren nach Anspruch 1, wobei das Segment des Primers 5 bis 50 Nukleotide, insbesondere 10 bis 40 Nukleotide lang ist.

3. Verfahren nach Anspruch 2, wobei das Bindungselement des Primers ein Oligonukleotid von 6 bis 50 Nukleotiden, insbesondere 10 bis 40 Nukleotide lang ist und zu keinem Teil der Ziel-Nukleinsäure komplementär ist.

4. Verfahren nach Ansprüchen 2 oder 3, wobei das Bindungselement des Primers ein Peptid ist.

5. Verfahren zum Detektieren eines Polymorphismus in einer Ziel-Nukleinsäure, umfassend:
Bereistellen einer Ziel-Nukleinsäure einschließend eine erste Base an einer Position, die vermutlich einen Polymorphismus aufweist, und eine zweite Base unmittelbar 3' zu der ersten Base,
Vermehren der Zielnukleinsäure mit einem ersten Primer und einem zweiten Primer, wobei der erste Primer ein erstes Nukleotid umfasst, das an dem 3'-Terminus vorliegt und eine Base beinhaltet, die zu der ersten Base der Ziel-Nukleinsäure komplementär ist, ein zweites Nukleotid, das unmittelbar 5' zu dem ersten Nukleotid liegt und eine Base beinhaltet, die zu der zweiten Base der Ziel-Nukleinsäure nicht komplementär ist, ein Segment von Nukleotiden, das unmittelbar 5' zu dem zweiten Nukleotid vorliegt und zu einem Teil der Ziel-Nukleinsäure komplementär ist, der unmittelbar 3' zu der zweiten Base vorliegt, und ein erstes Bindungselement eines spezifischen Bindungspaares, das an den 5'-terminus des Segmentes kovalent gebunden ist,
in Kontakt bringen der vermehrten Ziel-Nukleinsäure mit einem zweiten Bindungselement des spezifischen Bindungspaares, und
Detektieren der vermehrten Ziel-Nukleinsäure, die an das zweite Bindungselement bindet.

6. Verfahren nach Anspruch 5 weiter umfassend, Vermehren der Ziel-Nukleinsäure in der Anwesenheit eines zweiten ersten Primers, wobei das erste Nukleotid in einem der zwei ersten Primer mutiert und das erste Nukleotid in dem anderen als der Wildtyp vorliegt.

7. Verfahren nach Ansprüchen 5 oder 6, wobei das zweite Bindungselement an einem festen Träger immobilisiert vorliegt.

8. Verfahren nach Ansprüchen 5 bis 7, wobei der zweite Primer an dem 5'-Terminus eine Markierung beinhaltet.

9. Verfahren nach Ansprüchen 5, 6 oder 7, wobei jedes der ersten Bindungselemente der ersten Primer unabhängig ein Peptid oder ein Oligonukleotid ist, das zu keinem Teil der Ziel-Nukleinsäure komplementär ist.

10. Verfahren nach Ansprüchen 5, 6, 7 oder 9, wobei die ersten Bindungselemente der ersten Primer unterschiedliche Oligonukleotide sind.

11. Verfahren nach Ansprüchen 5 oder 10, wobei das erste Bindungselement ein Oligonukleotid ist, das zu keinem Teil der Ziel-Nukleinsäure komplementär ist.

12. Verfahren nach Ansprüchen 5 oder 10, wobei das erste Bindungselement als ein Peptid vorliegt.

13. Verwendung eines Kits in einem Verfahren gemäß einem der vorstehenden Ansprüche zum Vermehren einer Ziel-Nukleinsäure.

## Revendications

1. Une méthode pour amplifier un acide nucléique cible qui inclut une première base en une position suspectée d'un polymorphisme et une seconde base immédiatement en 3' de la première base comprenant l'utilisation d'une amorce de discrimination, l'amorce comprenant :
un premier nucléotide qui est localisé au terminus 3' de l'amorce et qui contient une base qui est complémentaire de la première base de l'acide nucléique cible ;
un second nucléotide qui est localisé immédiatement en 5' du premier nucléotide et qui contient une base qui n'est pas complémentaire de la seconde base de l'acide nucléique cible ;
un segment de nucléotides qui est localisé immédiatement en 5' du second nucléotide et qui est complémentaire d'une partie de l'acide nucléique cible et qui est immédiatement en 3' de la seconde base ; et
un membre de liaison d'une paire de liaison spécifique relié par covalence au terminus 5' du segment.

2. La méthode de la revendication 1 dans laquelle le segment de l'amorce est long de 5 à 50 nucléotides, en particulier de 10 à 40 nucléotides.

3. La méthode de la revendication 2 dans laquelle le membre de liaison de l'amorce est un oligonucléotide long le 6 à 50 nucléotides, en particulier de 10 à 40 nucléotides et non complémentaire d'aucune partie de l'acide nucléique cible.

4. La méthode de la revendication 2 ou 3 dans laquelle le membre de liaison de l'amorce est un peptide.

5. Une méthode pour détecter un polymorphisme dans un acide nucléique cible comprenant :
fournir un acide nucléique cible incluant une première base en une position suspectée d'un polymorphisme et une seconde base immédiatement en 3' de la première base ; amplifier l'acide nucléique cible avec une première amorce et une seconde amorce, dans laquelle la première amorce comprend un premier nucléotide qui est localisé au terminus 3' et qui contient une base qui est complémentaire de la première base de l'acide nucléique cible, un second nucléotide qui est localisé immédiatement en 5' du premier nucléotide et qui contient une base qui n'est pas complémentaire de la seconde base de l'acide nucléique ; un segment de nucléotides qui est localisé immédiatement en 5' du second nucléotide et qui est complémentaire d'une partie de l'acide nucléique cible et qui est immédiatement en 3' de la seconde base ; et un premier membre de liaison d'une paire de liaison spécifique relié par covalence au terminus 5' du segment ;
contacter l'acide nucléique cible amplifié avec un second membre de liaison de la paire de liaison spécifique ; et
détecter l'acide nucléique cible amplifié qui se lie au second membre de liaison.

6. La méthode de la revendication 5 qui comprend en outre d'amplifier l'acide nucléique cible en présence d'une seconde première amorce, dans laquelle le premier nucléotide dans l'une des deux premières amorces est mutant et le premier nucléotide dans l'autre est de type sauvage.

7. La méthode de la revendication 5 ou 6 dans laquelle le second membre de liaison est immobilisé sur un support solide.

8. La méthode des revendications 5 à 7 dans laquelle la seconde amorce contient une étiquette au terminus 5'.

9. La méthode de la revendication 5, 6 ou 7 dans laquelle chacun des premiers membres de liaison des premières amorces est, indépendamment, un peptide ou un oligonucléotide non complémentaire d'aucune partie de l'acide nucléique cible.

10. La méthode des revendications 5, 6, 7 ou 9 dans laquelle les premiers membres de liaison des premières amorces sont des oligonucléotides différents.

11. La méthode des revendications 5 ou 10 dans laquelle le premier membre de liaison est un oligonucléotide non complémentaire d'aucune partie de l'acide nucléique cible.

12. La méthode des revendications 5 ou 10 dans laquelle le premier membre de liaison est un peptide.

13. Utilisation d'une trousse dans une méthode selon l'une des revendications précédentes pour amplifier un acide nucléique cible.
